# EUROPEAN PATENT SPECIFICATION

(11) **EP 2 080 438 B1**
(45) Date of publication and mention of the grant of the patent: **30.03.2011**
(21) Application number: 08460002.2
(22) Date of filing: 17.01.2008
(51) Int. Cl.: A23L 1/304, A61K 33/16

(54) **Natural mineral water for use in prophylaxis, hygiene and treatment of teeth and paradontium disease**
Naturmineralwasser zur Prophylaxe, Hygiene und Behandlung von Zahn- und Zahnfleischerkrankungen
L'eau minérale naturelle pour la prévention, l'hygiène et le traitement des maladies dentaires et péridentaires

(43) Date of publication of application: 22.07.2009
(73) Proprietor: Cieslik, Tadeusz, 41-800 Zabrze (PL); Nocon, Jacek, 40699 Erkrath (DE); Rauch, Jan, 34-100 Wadowice (PL)
(72) Inventor: Cieslik, Tadeusz, 41-800 Zabrze (PL); Nocon, Jacek, 40699 Erkrath (DE); Rauch, Jan, 34-100 Wadowice (PL)
(74) Representative: Gizinska-Schohe, Malgorzata

(56) References cited:
- RU-C2- 2 272 637
- US-A- 3 887 482
- US-A- 5 846 522
- ANONYMOUS: "Aktuelle Empfehlungen zur Kariesvorbeugung mit Fluoriden" INTERNET ARTICLE, [Online] 17 June 2007 (2007-06-17), XP002485078 Retrieved from the Internet: URL:http://web.archive.org/web/20070617220 834/http://babyernaehrung.de/fluorid-gabe. htm> [retrieved on 2008-06-19]

## Description

The subject of the invention is natural mineral water for use in prophylaxis, hygiene and treatment of teeth and paradontium diseases. This water can be used in all kinds of paradontopathy and it can also be used to prevent dental caries, and to stop development of existing dental caries.

Nowadays in everyday hygiene procedures, including oral hygiene procedures, we commonly use chemically conditioned water from water-pipe network or directly from natural surface and deep water intakes. Almost all the above mentioned sources of water, with few exceptions, can pose biological and chemical threats to our oral health. Although European Union standards are rather strict, they allow the presence of chemical compounds which increase acidity and some pathogenic bacterial colonies in the first class cleanliness.

This invention aims at using appropriate, cheap mineral water in everyday oral hygiene procedures.

Spring water acknowledged as mineral water, in various surface water and ground water reservoirs and natural water bodies existing on various continents has been examined. The research aimed at finding water which would be suitable for usage in everyday hygiene procedures, and which could help to treat illnesses of oral cavity, and to prevent new oral cavity illnesses like those enumerated at the beginning.

Unexpectedly natural mineral water has been discovered. Its physical, chemical and biological properties can efficiently help to solve the above mentioned problems.
The properties of natural mineral water are as follows :
1. pH - from 7,6 to 8,1
2. Electrolytic conductivity - from 180 to 210 µS/cm at 20°C
3. Hardness - from 110 to 150 mg CaCO₃/L
4. Chlorides - from 0,5 to 2,5 mg Cl/l
5. Fluorides - from 0,01 to 0,03 mg F/l
6. Sodium - from 0,6 to 1,6 mg Na/l
7. Potassium - from 1,2 to 2,2 mg K/l
8. Calcium - from 30 to 40 mg Ca/l
9. Magnesium - from 5 to 14 mg Mg/l
10. Faecal coli bacteria (thermotolerant) - 0 in 100 ml
11. Coli bacteria - 0 in 100 ml
12. Enterococcus (Streptococcus faecalis) - 0 in 100 ml
13. Clostridium perfrigens - 0 in 100 ml
14. Total number of bacteria at 37°C after 24 hours - from 1 to 2 in 1 ml
15. Total number of bacteria at 22°C after 72 hours - from 3 to 5 in 1 ml

Biological, chemical and/or physical properties of the above components of the water are described below :
**Re para 1/p** According to the research conducted for years in most cases reaction pH in oral cavity is from 5 to 7,5. Hence the reaction is acid or slightly above neutral level. Such pH environment creates favourable conditions for the growth of microorganisms, mainly Streptococcus and Actinomycaes, which cause dental caries. The lower pH level, the more pathogenic bacteria in dental plaque and the higher is the risk of dental caries incidence. Paradontium diseases also develop in the above described pH environment. Microorganisms Actinobacillus, Porphyromonas, Bacteroides, Fusobacterium, Peptostreptococcus and Treponema, which cause paradontium diseases, develop well in acid environment - pH<7. Pathogenic bacteria colonies in oral cavity could be reduced by introduction of basic environment. Mineral water which is the essence of this invention fulfills this task.
**Re para 2/p .** Electrolytic conductivity level depends on the amount of carbohydrates and sulfates in the substance examined. The more carbohydrates and sulfates there are in the solution, the higher are acidity and conductivity. As it is mentioned in point re para 1/p the essence of the invention is reduction of acidity in oral cavity and introduction of basic environment. The standard for the I class cleanliness is 400 µS/cm at 20°C. At this level of conductivity water's pH is about 6,5. Water, according to the invention, has electrolytic conductivity in range of 180 to 210 µS/cm at 20°C, and due to this it increases the pH of the oral cavity environment up to the value in range of 7,6 to 8,1.
**Re para 3/p** Hardness of water determines the level of CaCO₃ in 1000 ml. The standard for I class of cleanliness is maximum 250mg CaCO₃/l. The lower is the water hardness level, the better is action of detergents contained in toothpastes. Low level of harness facilitates dissolution of carbohydrates and fats which are medium for pathogenic microorganisms in oral cavity. Preparation facilitates cleaning of teeth surface and mucous membrane in oral cavity and improves action of detergents (tensides) contained in toothpastes. Amount of foam created during tooth brushing is an additional organoleptic indication. The more foam is created during cleaning an oral cavity with solution of water and detergent (toothpaste) the softer water becomes, therefore cleaning procedure is more effective. Water, according to this invention fulfills this task.
**Re para 4/p** Chlorides - The standard for I class cleanliness is 25mg Cl/l. The water according to the invention, contains trace amounts below allowable values.
**Re para 5/p** Fluorides - The standard for I class cleanliness is 0,5mg F/l. The water according to the invention, contains trace amounts below allowable values.
**Re para 6/p** Sodium - The standard for I class cleanliness is 60mg Na/l. The water according to the invention, contains trace amounts below allowable values.
**Re para 7/p** Potassium - The standard for I class cleanliness is 10mg K/l. The water according to the invention, contains trace amounts below allowable values.
**Re para 8/p** Calcium - The standard for I class cleanliness is 50mg Ca/l. The water according to the invention, contains trace amounts below allowable values.
**Re para 9/p** Magnesium - The standard for I class cleanliness is 25mg Mg/l. The water according to the invention, contains trace amounts below allowable values.
**Re para 10-15/p** Water according to the invention, does not contain any pathogenic microorganisms, therefore it does not cause any infections.

So far no action has been taken to put into use water whose compositions is suitable for prophylaxis of oral cavity. Prophylactic action of the water according to this invention will also be effective in case of using it for fixed prostheses. The water will also help to maintain oral cavity health at high level. Using cheap water whose composition is strictly defined (as above) by a large number of customers and patients will contribute towards the objectivization of scientific research within the scope of prophylaxis of oral cavity and conducting this research among big populations. Therefore use of the water will improve public health and reduce health care expenses. Application of the water, according to the invention, not only brings the benefits enumerated above, but also increases motivation to take better care of oral cavity hygiene. It should also be stressed that this water complies with I class cleanliness requirements and is fit for consumption.

Common and regular use of the water defined according to the invention allows to maintain high standards of oral cavity hygiene due to the following four factors:
- increasing the pH of oral cavity environment results in reducing the number of pathogenic microorganisms whose multiplication is stopped,
- supporting action of detergents contained in toothpastes through effective emulsification of carbohydrates and fats contained in dental plaque,
- reduction of the amount of tartar deposited on teeth due to presence of trace amounts of metals which are components of the tartar,
- elimination of all additional sources of infection due to lack of pathogenic microorganisms.

## Claims

1. Natural mineral water for use in everyday oral hygiene procedures in the prophylaxis and treatment of teeth and paradontium diseases **characterized in** the following physiochemical and biological properties: pH 7,6 - 8,1; electrolytic conductivity 180-210 µS/cm at 20°C, hardness 110 - 150 mg CaCO₃/l; chlorides 0,5 - 2,5 mg Cl/l; fluorides 0,01 - 0,03 mg F/l; sodium 0,6 - 1,6 mg Na/l; potassium 1,2 - 2,2 mg K/l; calcium 30 - 40 mg Ca/l; magnesium 5 - 14 mg Mg/l; faecal coli bacteria (thermotolerant) 0 in 100 ml; coli bacteria 0 in 100 ml; enterococcus (Streptococcus faecalis) 0 in 100 ml; clostridium perfrigens 0 in 100 ml; total number of bacteria at 37°C after 24 hours from 1 to 2 in 1 ml and total number of bacteria at 22°C after 72 hours from 3 to 5 in 1 ml.

2. Natural mineral water according to claim 1 for use in the prophylaxis and treatment of paradontopathy.

3. Natural mineral water according to claim 1 for use in the prophylaxis and treatment of dental caries.

4. Natural mineral water for use in everyday oral hygiene procedures in the hygiene of teeth and paradontium **characterized in** the following physiochemical and biological properties: pH 7,6 - 8,1; electrolytic conductivity 180 - 210 µS/cm at 20°C, hardness 110 - 150 mg CaCO₃/l; chlorides 0,5 - 2,5 mg Cl/l; fluorides 0,01 - 0,03 mg F/l; sodium 0,6 - 1,6 mg Na/l; potassium 1,2 - 2,2 mg K/l; calcium 30 - 40 mg Ca/l; magnesium 5 - 14 mg Mg/l; faecal coli bacteria (thermotolerant) 0 in 100 ml; coli bacteria 0 in 100 ml; enterococcus (Streptococcus faecalis) 0 in 100 ml; clostridium perfrigens 0 in 100 ml; total number of bacteria at 37°C after 24 hours from 1 to 2 in 1 ml and total number of bacteria at 22°C after 72 hours from 3 to 5 in 1 ml.

## Patentansprüche

1. Natürliches Mineralwasser zur Verwendung bei täglichen Mundhygienemaßnahmen bei der Prophylaxe und Behandlung von Erkrankungen der Zähne und des Parodontiums, **gekennzeichnet durch** die folgenden physikalisch-chemischen und biologischen Eigenschaften: pH 7,6 - 8,1, elektrolytische Leitfähigkeit 180 - 210 µS/cm bei 20°C, Härte 110 - 150 mg CaCO₃/l, Chloride 0,5 - 2,5 mg Cl/l, Fluoride 0,01 - 0,03 mg F/l, Natrium 0,6 - 1,6 mg Na/l, Kalium 1,2 - 2,2 mg K/l, Calcium 30 - 40 mg Ca/l, Magnesium 5-14 mg Mg/l, fäkale Coli-Bakterien (thermotolerant) 0 in 100 ml, Coli-Bakterien 0 in 100 ml, Enterokokken (Streptococcus faecalis) 0 in 100 ml, Clostridium perfrigens 0 in 100 ml, Anzahl Bakterien insgesamt bei 37°C nach 24 Stunden: 1 bis 2 in 1 ml und Anzahl Bakterien insgesamt bei 22°C nach 72 Stunden: 3 bis 5 in 1 ml.

2. Natürliches Mineralwasser nach Anspruch 1 zur Verwendung bei der Prophylaxe und Behandlung von Parodontopathie.

3. Natürliches Mineralwasser nach Anspruch 1 zur Verwendung bei der Prophylaxe und Behandlung von Zahnkaries.

4. Natürliches Mineralwasser zur Verwendung bei täglichen Mundhygienemaßnahmen für die Hygiene der Zähne und des Parodontiums, **gekennzeichnet durch** die folgenden physikalisch-chemischen und biologischen Eigenschaften: pH 7,6 - 8,1, elektrolytische Leitfähigkeit 180 - 210 µS/cm bei 20°C, Härte 110 - 150 mg CaCO₃/l, Chloride 0,5 - 2,5 mg Cl/l, Fluoride 0,01 - 0,03 mg F/l, Natrium 0,6 - 1,6 mg Na/l, Kalium 1,2 - 2,2 mg K/l, Calcium 30 - 40 mg Ca/l, Magnesium 5 - 14 mg Mg/l, fäkale Coli-Bakterien (thermotolerant) 0 in 100 ml, Coli-Bakterien 0 in 100 ml, Enterokokken (Streptococcus faecalis) 0 in 100 ml, Clostridium perfrigens 0 in 100 ml, Anzahl Bakterien insgesamt bei 37°C nach 24 Stunden: 1 bis 2 in 1 ml und Anzahl Bakterien insgesamt bei 22°C nach 72 Stunden: 3 bis 5 in 1 ml.

## Revendications

1. Eau minérale naturelle pour utilisation dans le cadre de procédures d'hygiène orale quotidienne dans la prophylaxie et le traitement de maladies dentaires et du parodontium, **caractérisée par** les propriétés physiochimiques et biologiques suivantes : pH 7,6 - 8,1 ; conductivité électrolytique 180 - 210 µS/cm à 20°C, dureté 110 - 150 mg CaCO₃/l ; chlorures 0,5 - 2,5 mg Cl/l; fluorures 0,01 - 0,03 mg F/l sodium 0,6 - 1,6 mg Na/l ; potassium 1,2 - 2,2 mg K/l ; calcium 30 - 40 mg Ca/l; magnésium 5 - 14 mg Mg/l bactéries fécales antibiorésistances (thermotolérantes) 0 dans 100 ml ; bactéries coli 0 dans 100 ml ; entérocoques (Streptococcus faecalis) 0 dans 100 ml ; clostridium perfrigens 0 dans 100 ml ; nombre total de bactéries à 37°C après 24 heures de 1 à 2 dans 1 ml et nombre total de bactéries à 22°C après 72 heures de 3 à 5 dans 1 ml.

2. Eau minérale naturelle selon la revendication 1, pour utilisation dans la prophylaxie et le traitement de la parodontopathie.

3. Eau minérale naturelle selon la revendication 1, pour utilisation dans la prophylaxie et le traitement de caries dentaires.

4. Eau minérale naturelle pour utilisation dans le cadre de procédures d'hygiène orale quotidienne dans l'hygiène des dents et du parodontium, **caractérisée par** les propriétés physiochimiques et biologiques suivantes : pH 7,6 - 8,1 ; conductivité électrolytique 180-210 µS/cm à 20°C, dureté 110 - 150 mg CaCO₃/l ; chlorures 0,5 - 2,5 mg Cl/l ; fluorures 0,01 - 0,03 mg F/l ; sodium 0,6 - 1,6 mg Na/l ; potassium 1,2 - 2,2 mg K/l ; calcium 30 - 40 mg Ca/l ; magnésium 5 - 14 mg Mg/l ; bactéries fécales antibiorésistances (thermotolérantes) 0 dans 100 ml ; bactéries coli 0 dans 100 ml ; entérocoques (Streptococcus faecalis) 0 dans 100 ml ; clostridium perfrigens 0 dans 100 ml ; nombre total de bactéries à 37°C après 24 heures de 1 à 2 dans 1 ml et nombre total de bactéries à 22°C après 72 heures de 3 à 5 dans 1 ml.
